# EUROPÄISCHE PATENTSCHRIFT

(11) **EP 2 224 878 B1**
(45) Veröffentlichungstag und Bekanntmachung des Hinweises auf die Patenterteilung: **20.07.2011**
(21) Anmeldenummer: 08854450.7
(22) Anmeldetag: 27.11.2008
(51) Int. Cl.: A61F 2/01, A61F 2/00

(54) **VORRICHTUNG ZUM FILTERN VON BLUT**
DEVICE FOR FILTERING BLOOD
DISPOSITIF DE FILTRATION DU SANG

(30) Priorität: 27.11.2007 DE 102007056946
(43) Veröffentlichungstag der Anmeldung: 08.09.2010
(73) Patentinhaber: Pah, Gunnar, 1322 Hørvik (NO)
(72) Erfinder: Pah, Gunnar, 1322 Hørvik (NO)
(74) Vertreter: Ganahl, Bernhard
(86) Internationale Anmeldenummer: PCT/EP2008/066297
(87) Internationale Veröffentlichungsnummer: WO 2009/068596

(56) Entgegenhaltungen:
- WO-A-2007/135065
- US-A1- 2006 173 490
- US-B1- 6 361 545
- US-B1- 6 499 487

## Beschreibung

Die vorliegende Erfindung betrifft eine Vorrichtung zum Filtern von Blut. Der Filter ist zum Filtern des im Aortabogen abzweigenden Blutes zur Versorgung des Gehirns und der Arme vorgesehen.

In der nachveröffentlichten deutschen Patentanmeldung DE 10 2006 024 179 bzw. der WO 2007/135065 A2 ist eine Vorrichtung zum Filtern von Blut beim Beseitigen einer Herzklappenstenose beschrieben. Diese umfasst einen Filterkatheter, welcher zum Filtern des stromabwärtig hinter einer Herzklappe befindlichen Blutes an seinem distalen Ende einen Filter aufweist. Der Filter ist aus dem Filterkatheter distal ausschiebbar und in diesen hineinschiebbar. Der Filter ist derart radial aufweitend ausgebildet, dass er sich im ausgefahrenen Zustand vom Filterkatheter radial nach außen erstreckt und an einer Blutgefäßwandung anliegt. Der Filterkatheter weist ein Katheterlumen auf, das derart ausgebildet ist, dass ein Ballonkatheter durch das Katheterlumen hindurch geführt werden kann. Mit dem Ballonkatheter kann die Herzklappe gesprengt werden, wobei der stromabwärts angeordnete Filter hierbei anfallendes Plaque, Debris und Embolie auslösendes Material aus dem Blut filtert.

Aus der WO 99/23976 ist ein Katheter mit einem zusammenlegbaren Filterelement bekannt. Das Filterelement, ist auf einem Filterträger angeordnet und zum Zuführen durch ein Blutgefäßsystem des Patienten ausgebildet. Das Filterelement ist von einer zusammengelegten Lagerposition im Filterträger, für die Bewegung durch das Gefäßsystem, in eine entfaltete Position überführbar, wobei in der entfalteten Position ein Blutgefäß abgesperrt wird, so dass Blut, welches durch das Blutgefäß strömt, durch das Filterelement geführt wird. Das Filterelement umfasst einen zusammenlegbaren Filterkörper mit einem Einlassende und einem Auslassende, wobei das Einlassende des Filterkörpers eine oder mehrere Öffnungen besitzt, die Blut, dem eine Embolie auslösendem Material, Plaque und Debris erlaubt, in den Filterkörper einzutreten.-Das Auslassende des Filterkörpers weist eine Mehrzahl von Auslassöffnungen auf, die derart ausgebildet sind, dass Blut hindurchtreten kann, aber unerwünschtes embolisches Material innerhalb des Filterkörpers zurückbehalten wird. Weiterhin sind Mittel vorgesehen, um die Einlassöffnung und die Auslassöffnung des Filterkörpers zu schließen. Diese Öffnungen werden geschlossen, bevor der Filter zum Herausziehen desselben geschlossen wird. Diese Druckschrift schlägt hierfür eine Vielzahl unterschiedlicher Filterelemente vor.

Aus der WO 00/67668 ist ein weiteres Filterelement bekannt mit einem zusammenlegbaren Filterkörper. Der Filterkörper ist von einer zusammengefalteten Position in der er durch ein Gefäßsystem bewegt werden kann in eine ausgefaltete Position, um sich in einem Blutgefäß zu erstrecken, überführbar. In der ausgefalteten Position wird Blut, welches durch das Blutgefäß strömt, durch das Filterelement geführt und gefiltert. Ein distaler Einlassbereich des Filterkörpers besitzt einen oder mehrere Einlassöffnungen, die so bemessen sind, dass Blut, eine Embolie verursachendes Material, Plaque und Debris in den Filterkörper eintreten können und ein proximalen Ausgangsbereich, der viele kleine Auslassöffnungen besitzt, die so bemessen sind, dass Blut hindurchtreten kann, aber embolisches Material, Plaque und Debris zurückgehalten werden. Der Filterkörper ist zumindest teilweise eine Laminatkonstruktion, aufweisend eine Membran, die mit einer Beschichtung versehen ist, die biokompatibel ist.

Aus der US 6,676,683 B1 geht ein Filterkatheter hervor, der an seinem Endbereich einen ausfahrbaren und aufweitbaren Filter aufweist. Der Katheter selbst ist ein Drahtkatheter, an dem weitere Katheter, die diesen Drahtkatheter umfassen, entlang geführt werden können, um in den Blutgefäßen entsprechend positioniert zu werden.

Aus der EP 980 278 B1 geht ein ähnlicher Katheter mit einem Führungsdraht hervor, an dessen Ende ein expandierbares Filterelement angeordnet ist.

Nachteilig bei den aus dem Stand der Technik bekannten Filtern ist, dass sie den Durchfluss des Blutes beeinflussen und einen erheblichen Rückstau im Blutgefäß erzeugen.

Eine künstliche Aortaklappe ist z. B. aus der EP 1 335 683 B1 bekannt. Weitere implantierbare Herzklappenprothesen sowie Katheter für die Implantation einer solchen Herzklappenprothese gehen aus der EP 592 410 B1, der US 2004/0210304 A1, US 7,018,406 B2, WO2006/127765 A1, der US 2003/0036795 A1, der US 5,411,552, der US 6,168,614 B1, der US 6,582,462 B1 und der WO91/17720 hervor.

Aus der DE 1 988 277 T1 ist ein Katheter mit einem zusammenfaltbaren Filterkörper bekannt. Der Filterkörper ist aus einem distalen Ende des Katheters ausschiebbar. Der Filterkörper weist einen proximalen Einlass auf, der sich vom Katheter zu einem Filter hin trichterförmig aufweitet. Der Einlass weist mehrere Einlassöffnungen auf, durch die Blut und Emboliematerial in das Filterelement eindringen können. Der Filterkörper umfasst einen rohrförmigen Abschnitt mit einem distalen Auslass. Der Auslass weist mehrere Auslassöffnungen auf, die derart ausgebildet sind, dass Blut hindurchströmt und unerwünschtes Emboliematerial innerhalb des Körpers des Filterelements zurückgehalten wird. Die proximalen Einlässe sind größer als die distalen Auslässe.

In der WO 03/017823 A2 ist ein Filterkatheter bekannt der in Strömungsrichtung in eine Arterie eingebracht wird. Der Filter weist ebenfalls einen rohrförmigen und einen sich trichterförmig verjüngenden Abschnitt auf. Der sich verjüngende Abschnitt ist am distalen Ende des Filters ausgebildet und der rohrförmige Abschnitt am proximalen Ende des Filters. Der proximale Abschnitt weist Öffnungen auf, durch die unerwünschtes Material in den Filter eindringen kann

Aus der WO 2006/116636 geht ein selbst expandierender Stent mit Filterkörper hervor. Der Filterkörper kann im distalen Endbereich des Stents ausgebildet sein und weist an seinem distalen Ende Filterporen auf, die es ermöglichen, dass Blut durch sie hindurchtritt aber Embolie verursachendes Material oder größere Partikel aufgehalten werden.

In der WO 2007/06751 ist eine implantierbare medizinische Vorrichtung beschrieben. Die Vorrichtung weist einen rohrförmigen Abschnitt, der als Stent ausgebildet ist, und einen trichterförmigen Abschnitt, der als Filter ausgebildet ist, auf. Die Vorrichtung kann in beiden Längsrichtungen von Blut durchströmt werden und mit oder entgegen der Strömungsrichtung des Blutes in den Körper eingebracht werden.

In der WO 2007/133887 A1 ist ein "fingerhutartig" ausgebildeter Filterkatheter beschrieben der in Strömungsrichtung des Blutes in die Arterie eingebracht wird. Der Filter kann über seine gesamte Länge gleich große Filteröffnungen aufweisen.

In der US 2007/0203559 A1 ist ein Stent beschrieben der ein proximales Ende und ein distales Ende aufweist. Der Stent besitzt eine Art Glockenform, mit einem aufgeweiteten distalen Ende und einem trichterförmigen proximalen Ende.

Aus der US 6,361,545 B1 geht ein Filterkatheter hervor, der einen äußeren Schlauch und einen Katheterschaft aufweist. Auf dem Katheterschaft ist eine Filterstützstruktur befestigt, auf der ein Filternetz befestigt ist. Am stromaufwärts angeordneten Ende weist das Filternetz kleinere Filtermaschen als am stromabwärts angeordneten Ende auf. Mit den feineren Maschen sollen sowohl Mikroembolien als auch Makroembolien gefiltert werden, wohingegen mit den gröberen Maschen lediglich Makroembolien gefiltert werden sollen.

Der Erfindung liegt die Aufgabe zu Grunde eine Vorrichtung und ein Verfahren zum Filtern von Blut zu schaffen, mit welchen Embolie verursachendes Material, Plaque und Debris sicher aufgehalten und der Einfluss des Filters auf den Durchfluss des Blutes sowie der sich daraus ergebende Rückstau möglichst gering gehalten wird.

Die Aufgabe wird mit einer Vorrichtung mit den Merkmalen des Anspruchs 1 gelöst. Vorteilhafte Ausgestaltungen sind in den entsprechenden Unteransprüchen angegeben.

Die erfindungsgemäße Vorrichtung zum Filtern von Blut, umfasst einen Filterkatheter welcher zum Filtern des stromabwärts hinter der Herzklappe in die Arterien zur Versorgung des Gehirns und der Arme abzweigenden Blutes an seinem distalen Ende einen Filter aufweist, welcher aus einer Gitterstruktur ausgebildet ist und aus einem Katheterschlauch distal ausschiebbar ist, und der Filter im ausgeschobenen Zustand in Richtung von proximal nach distal aufweitend ausgebildet ist, wobei der Filter einen distalen Filterabschnitt und einen proximalen Abschnitt aufweist, und der distale Filterabschnitt Filteröffnungen aufweist und der proximale Abschnitt eine oder mehrere Öffnungen aufweist, die größer als die Filteröffnungen des distalen Filterabschnitts ausgebildet sind, und die Gitterstruktur einen distalen Endbereich zum dichten Anliegen an der aufsteigenden Aorta aufweist, wobei zumindest der distale Endbereich des distalen Filterabschnitts mit einer höheren Steifigkeit in radialer Richtung als der restliche Filterabschnitt ausgebildet ist.

Durch das Vorsehen größerer Öffnungen am proximalen Abschnitt wird der Rückstau gering gehalten. Hierdurch wird das kranke Herz weniger belastet. Weiterhin wird die Kraft, die die Blutströmung auf den Filter ausübt, gering gehalten, wodurch ein unbeabsichtigtes Verschieben des distalen Endes Filters in Strömungsrichtung vermieden wird.

Es hat sich gezeigt, dass trotz einer fast vollständigen Öffnung des proximalen Abschnittes ausreichend Blut im Aortabogen gefiltert und zum Gehirn abgezweigt werden kann. Weiterhin wird durch die größeren Öffnungen im proximalen Abschnitt eine ausreichende Versorgung des restlichen Körpers sichergestellt, auch wenn die Funktionstätigkeit des Herzens eingeschränkt ist.

Der Abschnitt im Aortabogen, in dem die Arterien zur Versorgung des Gehirns und der Arme abzweigen, wird im folgenden als Aortabogenabschnitt bezeichnet. Die Arterien zur Versorgung des Gehirns und der Arme sind der Truncus brachiocephalicus, der sich in die rechte Schlüsselbeinarterie (Arteria subclavia dextra) und in die rechte gemeinsame Halsschlagader (Arteria carotis communis dextra) aufteilt, die linke gemeinsame Halsschlagader (Arteria carotis communis sinistra) und die linke Schlüsselbeinarterie (Arteria subclavia sinistra). Der Filter weist gemäß einer Ausführungsform einen röhrenförmigen distalen Filterabschnitt und einen proximalen Abschnitt auf, der sich vom distalen Filterabschnitt zum Katheterschlauch verjüngt. Der Filter ist länger als der Aortabogenabschnitt und derart ausgebildet, dass er im Bereich stromaufwärts vor dem Aortabogen zumindest an seinem distalen Endbereich dicht an der aufsteigenden Aorta anliegt. Im distalen Filterabschnitt sind Filteröffnungen ausgebildet. Der proximale Abschnitt weist eine oder mehrere Öffnungen auf, die größer als die Filteröffnungen des distalen Filterabschnitts sind.

Dadurch dass die Filteröffnungen des Filters im Bereich der Abzweigungen zu den Arterien zur Versorgung des Gehirns und der Arme relativ klein ausgebildet sind, können sehr feine Teilchen aus dem Blut gefiltert werden, wodurch verhindert wird, dass diese über die Arterien zur Versorgung des Gehirns und der Arme ins Gehirn gelangen und einen Gehirnschlag verursachen.

Mit dem proximalen Filterabschnitt werden gar keine bzw. nur größere Teilchen gefiltert. Kleinere Teilchen stellen in dem in die Beine fließenden Blut kein hoch relevantes Gesundheitsrisiko dar. Der Rückstau herkömmlicher Filterkatheter belastet hingegen das Herz erheblich, was bei derartigen Operationen äußerst problematisch ist.

Nach einer weiteren Ausführungsform der vorliegenden Erfindung umfasst die Vorrichtung einen Filterkatheter, welcher zum Filtern des stromabwärts hinter der Herzklappe im Aortabogen in die Arterien zur Versorgung des Gehirns und der Arme abzweigenden Blutes an seinem distalen Ende einen langgestreckten sich von distal nach proximal verjüngenden Filter aufweist. Der Filter geht an seinem proximalen Ende in einen Katheterschlauch über und ist aus dem Katheter distal ausschiebbar. Der Filter ist länger als der Aortabogenabschnitt, an dem die Arterien zur Versorgung des Gehirns und der Arme abzweigen und ist derart ausgebildet, dass er im Bereich stromaufwärts vor diesen zumindest mit seinem distalen Endbereich dicht an der aufsteigenden Aorta anliegt.

Durch die langgestreckte Ausbildung des Filters bzw. die Art der Anordnung im Bereich des Aortabogens ist eine große Filterfläche vorhanden. Durch die große Filterfläche des sich verjüngenden Filters wird der Rückstau des Blutes gering gehalten.

Der Filter ist derart ausgebildet sein, dass die Filteröffnungen des Filters von distal nach proximal, beispielsweise über drei Abschnitte, jeweils größer werden. Auf diese Weise wird sichergestellt, dass das in die Arterien zur Versorgung des Gehirns und der Arme abfließende Blut fein gefiltert wird. Durch die größeren Filteröffnungen am proximalen Ende des Filters wird der Durchfluss des Blutes kaum beeinträchtigt wird.

Nach einem weiteren Aspekt der Erfindung können die beiden oben beschriebenen Ausführungsformen auch mit Filteröffnungen ausgebildet sein, die im wesentlichen über die gesamte Länge des Filters eine etwa konstante Größe mit einem Durchmesser im Bereich von etwa 150 µm bis 250 µm und vorzugsweise 150 µm bis 200 µm aufweisen. Diese Öffnungsgröße ist sowohl ausreichend klein um Partikel aus dem zum Gehirn strömenden Blut zu filtern als auch noch groß genug um den Rückstau gering zu halten. Dies gilt insbesondere für die Ausführungsform des Filters, der sich allmählich von distal nach proximal verjüngt, da hier eine sehr große Filterfläche vorgesehen wird. Eine in Richtung zum proximalen Ende des Filters zunehmende Öffnungsgröße wird jedoch bevorzugt.

Bei Gebrauch wird der Filterkatheter ein Stück stromabwärts von der Herzklappe im Aortabogen im Bereich der Abzweigstellen der Arterien zur Versorgung des Gehirns und der Arme angeordnet.

Der Filterkatheter kann durch alle bekannten Zugangsweisen in den Körper eingeführt werden und durch die absteigende Aorta (Hauptschlagader) zum Aortabogen geführt werden. Beispielsweise wird der Filterkatheter durch die Femoral-Arterie oder die Radialis-Arterie oder die Subklavias-Arterie in den Körper eingeführt.

Der Filterkatheter ist derart ausgebildet, dass er entgegen der Strömungsrichtung des Blutes in die Aorta eingeführt werden kann.

Der Filter ist im ausgefahrenen Zustand derart ausgebildet, dass Embolie verursachendes Material, Plaque und Debris, das sich am Filter ansammelt, beim Einziehen des Filters in den Katheterschlauch mit in das Katheterlumen genommen wird und nicht in der Aorta verbleibt. Der Filter ist im Vergleich zu herkömmlichen Filtern wesentlich länger ausgebildet, wodurch er ein großes Volumen begrenzt, in dem sich zu filternde Teile ansammeln können. Beim Einziehen des Filters in den Katheterschlauch werden diese Teile mit in den Katheterschlauch eingezogen und sicher aus dem Körper entfernt.

Zum Ausfahren des Filters sind erfindungsgemäß unterschiedliche Mechanismen möglich. Der einfachste erfindungsgemäβe Mechanismus sieht derart aus, dass der Filter mit einem Filterdraht oder einer ähnlichen Vorrichtung verbunden ist und auf diese Weise aus dem Katheterschlauch geschoben bzw. in diesen hineingezogen wird. Zudem ist das Vorsehen eines Federelementes, welches entsprechend einwirkt, möglich.

Der Filter und/oder der Filterdraht können mit einer antitrombogenen Beschichtung versehen sein um ein Anhaften von Blut bzw. eine Gerinnung des Blutes zu verhindern.

Bei einer weiteren vorteilhaften Ausführungsform ist der Katheterschlauch aus zwei schlauchförmigen koaxialen Körpern ausgebildet, wobei der innere Körper fest mit dem Filter verbunden ist und durch Verschieben des Innenkörpers innerhalb des äußeren Körpers der Filter aus dem äußeren Körper aus- bzw. wieder in diesen eingefahren wird.

Der Katheterschlauch kann auch anstelle eines äußeren und eines inneren Körpers aus einem einteiligen, holen zylindrischen bzw. röhrenförmigen Körper ausgebildet sein. In der Wandung dieses Körpers sind vom proximalen zum distalen Ende verlaufende Hohlkanäle ausgebildet, in welchen jeweils ein Filterdraht gelagert ist. Am distalen Ende dieses Körpers ist eine Ausnehmung zur Aufnahme des Filters vorgesehen. Der Filter ist an seinem proximalen Ende an einem Ring befestigt. Der Ring ist wiederum an den Enden der Filterdrähte angeordnet. Mittels der Filterdrähte kann somit der Filter aus dem Katheterschlauch heraus geschoben werden und wieder in den Katheterschlauch hineingezogen werden. Die Filterdrähte werden alle gleichzeitig betätigt, damit ein Verkanten des Filters im Katheterschlauch vermieden wird. Vorzugsweise sind etwa drei bis fünf Filterdrähte vorgesehen, die voneinander jeweils im gleichen Winkel im Katheterschlauch beabstandet sind.

Der Durchmesser des Filterkatheters beträgt in etwa 8 bis 24 French (FR) (ca. 2,5 mm bis 8 mm), wobei die Einheit French eine in dem Bereich für Katheter verwendete übliche Größeneinheit ist.

Der Filter ist maximal auf einen Durchmesser von 120 French (ca. 40 mm) bis zu 180 French (ca. 60 mm) ausdehnbar. Der Aussendurchmesser, zumindest des distalen Endbereichs des Filters ist größer dimensioniert als die Aorta, um eine radiale Steifigkeit bzw., um eine Haftung durch Reib- und/oder Formschluss zu erzeugen damit der Filter in der Aorta fixiert wird.

Vorzugsweise erfolgt die Anordnung des Filters im Aortabogen an den Abzweigungen zu den Arterien zur Versorgung des Gehirns und der Arme. Durch die Filterung des in die Arterien zur Versorgung des Gehirns und der Arme abzweigenden Blutes wird vorteilhafterweise das Risiko eines Gehirnschlags vermindert.

Das Katheterlumen ist derart ausgebildet, dass ein Ballonkathteter hindurchgeführt werden kann. Die vorliegende Erfindung ist jedoch nicht auf die Durchführung eines Ballonkatheters beschränkt. Es können auch andere Katheter hindurchgeführt werden.

Der erfindungsgemäße Filterkatheter kann auch bei einer chirurgischen Perforation der aufsteigenden Aorta (siehe z.B. Fig. 4 der US 6,676,683 B1), bei der z.B. embolisches Material aus einem Blutgefäß enfernt werden soll, verwendet werden.

Es wird darüber hinaus auf die DE 10 2006 024 179 und die PCT/EP2007/054777 Bezug genommen.

Die Erfindung wird anhand der Zeichnungen beispielhaft erläutert. Es zeigen dabei schematisch:
- Figur 1: die Anordnung des erfindungsgemäßen Filterkatheters im Aortabogen,
- Figur 2: ein erstes Ausführungsbeispiel des Filterkatheters mit einem freigeschnittenen Katheterschlauch,
- Figur 3: ein zweites Ausführungsbeispiel des Filterkatheters mit einem freigeschnitte- nen Katheterschlauch,
- Figur 4: ein drittes Ausführungsbeispiel des Filterkatheters mit einem freigeschnittenen Katheterschlauch,
- Figur 5: ein viertes Ausführungsbeispiel des Filterkatheters mit einem freigeschnittenen Katheterschlauch,
- Figur 6: ein fünftes Ausführungsbeispiel des Filterkatheters mit einem freigeschnitte- nen Katheterschlauch,
- Figur 7: einen erfindungsgemäßen Filterkatheter zusammen mit einem Ballonkatheter in einer schematischen Schnittansicht,
- Figur 8: die Anordnung des Filterkatheters gemäß Fig. 4 im Aortabogen, und
- Figur 9: die Anordnung des Filterkatheters gemäß Fig. 5 im Aortabogen.

Die Erfindung wird anhand mehrerer Ausführungsbeispiele erläutert. Gleiche Teile sind mit gleichen Bezugszeichen versehen. Sofern nichts anderes ausgeführt ist, weisen sie die gleichen Eigenschaften wie bei den zuvor erläuterten Ausführungsbeispielen auf.

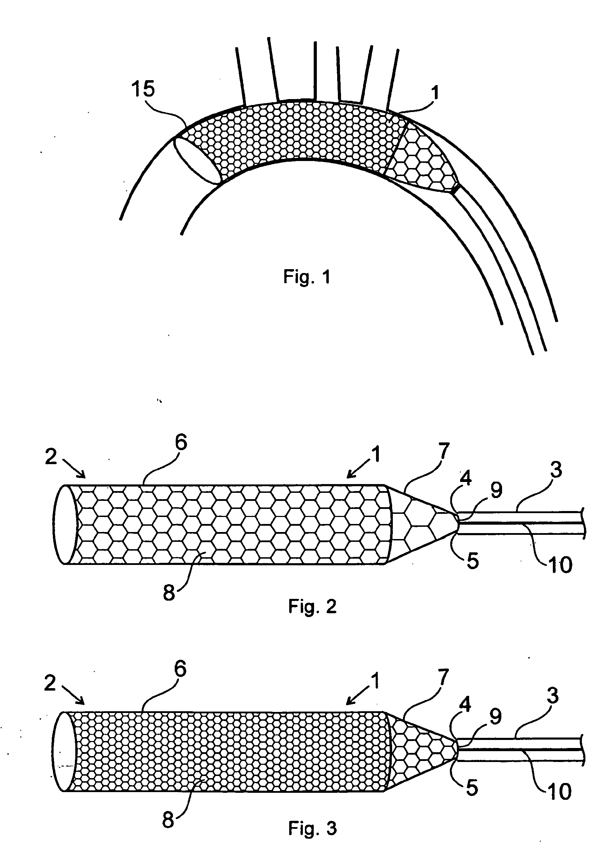

Die erfindungsgemäße Vorrichtung 1 ist ein Filterkatheter (Fig. 2-9). Der Filterkatheter 1 weist einen Filter 2 und einen Katheterschlauch 3 auf wobei der Filter 2 aus einem Katheterlumen 5 am distalen Ende 4 des Katheterschlauchs 3 ausschiebbar ausgebildet ist.

Der Filter 2 gemäß einem ersten Ausführungsbeispiel weist einen distalen Filterabschnitt 6, der hohlzylindrisch bzw. röhrenförmig ausgebildet ist, und einen proximalen Abschnitt 7 auf, der sich vom distalen Filterabschnitt zum Katheterschlauch 3 hin verjüngt (Fig. 2). Der distale Filterabschnitt 6 weist eine Länge von in etwa 3 cm bis 15 cm, bzw. eine Länge von in etwa 5 cm bis 14 cm, bzw. eine Länge von in etwa 6 cm bis 14 cm, bzw. eine Länge von in etwa 7 cm bis 14 cm, bzw. eine Länge von in etwa 8 cm bis 14 cm und vorzugsweise eine Länge von in etwa 10 cm bis 14 cm und einen Durchmesser im expandierten Zustand von in etwa 3 cm bis 6 cm auf. Der proximale Abschnitt 7 weist eine Länge von in etwa 3 cm bis 5 cm auf.

Im vorliegenden Ausführungsbeispiel ist der proximale Abschnitt 7 konusförmig ausgebildet. Er kann aber auch im Querschnitt konvex oder konkav gebogen ausgebildet sein oder eine andere sich verjüngende Form aufweisen.

Der Filter 2 ist aus einer Gitterstruktur aus einer Formgedächtnislegierung, wie z.B. Nitinol, oder einer anderen geeigneten Gedächtnislegierung, wie z.B. einer Eisenlegierung, einer Kupferlegierung, oder einem anderen Formgedächtnismaterial, wie z.B. Kunststoff, ausgebildet. Die Gitterstruktur ist derart ausgebildet, dass sie den Filter 2 beim Expandieren während des Ausschiebens aus dem Katheter 3 unterstützt. Das bedeutet, dass die Gitterstreben der Gitterstruktur im komprimierten Zustand im Katheterlumen 5 des Katheterschlauchs 3 unter Vorspannung sind. Beim Ausschieben aus dem Katheterlumen 5 entspannt sich die Gitterstruktur bzw. nimmt ihre vorgegebene Form ein und unterstützt auf diese Weise die Expandierung des Filters.

Es kann vorgesehen sein, den Filter vor dem Einbringen in den Körper zu kühlen, so dass dieser sich aufgrund der Körpertemperatur beim Ausschieben aus dem Katheterschlauch 3 erwärmt. Das Formgedächtnismaterial der Gitterstruktur nimmt hierdurch die expandierte Gestalt an und unterstützt somit die Entfaltung.

Die Gitterstruktur bzw. die Streben des Filters können mit einer antitrombogenen Beschichtung beschichtet sein, um ein Anhaften von Blut zu verhindern. Derartige Beschichtungen werden z.B. von der Firma SurModics/USA und der Firma Carmeda/Schweden hergestellt. Als Basisstoffe der antitrombogenen Beschichtung dienen z.B. Omega-3-Fettsäuren und Heparin.

Als Filterelement 8 ist eine Kunststofffolie vorgesehen, die z.B. aus Polyurethan (PU) oder einem anderen geeigneten Kunststoff ausgebildet ist. Die Folie ist auf der Innenseite der Gitterstruktur anliegend angeordnet und mit dieser verklebt. Die Poren des Filters weisen im distalen Filterabschnitt 6 einen geringeren Durchmesser als im proximalen Abschnitt 7 auf. Die Folie weist im Bereich des distalen Filterabschnitts 6 Poren mit einem Durchmesser von in etwa 100 µm bis 300 µm und vorzugsweise zwischen 120 µm und 200 µm auf und im proximalen Abschnitt 7 sind Poren mit einem Durchmesser von in etwa 150 µm bis 400 µm vorzugsweise zwischen 200 µm und 300 µm vorgesehen.

Die Kunststofffolie kann auch im Bereich zwischen den Maschen der Gitterstruktur oder außen an der Gitterstruktur angeordnet sein. Andere für das Filterelement 8 geeignete Materialien sind beispielsweise Teflon (PTFE), Polypropylen (PP) und Polyethylen (PE). Das Filterelement kann die Gitterstruktur des Filters auch vollständig umschließen, um einen Kontakt zwischen der Gitterstruktur und dem Blut zu verhindern. Auf diese Weise wird ein Anhaften bzw. eine Gerinnung des Blutes an der Gitterstruktur des Filters verhindert.

Es kann auch vorgesehen sein, dass der proximale Abschnitt 7 kein Filterelement 8 aufweist, sondern lediglich ca. 4 bis 10 umfänglich angeordnete Streben bzw. eine Gitterstruktur aufweist. Hierdurch kann der Hauptstrom des Blutes nahezu ungehindert fließen.

Bei einem gesunden Menschen werden zur Versorgung des Gehirns und der Arme etwa 20% des gesamten Blutstroms im Aortabogen abgezweigt. Die restlichen 80% fließen durch die absteigende Aorta weiter und versorgen den restlichen Körper.

Tests an Strömungsmodellen mit Blut haben gezeigt, dass bei einem Filter 2, der lediglich einen distalen Filterabschnitt 6 aufweist und ohne Filterelement 8 im proximalen Abschnitt 7 ausgebildet ist, eine ausreichende Blutmenge von annähernd 20% des gesamten Blutstroms über den distalen Filterabschnitt 6 in die Arterien zur Versorgung des Gehirns und der Arme gelangt. Der abzweigende Blutstrom kann also sehr fein gefiltert werden, trotzdem gelangt ausreichend Blut in die Arterien zur Versorgung des Gehirns und der Arme und dies sogar, wenn am proximalen Ende des Filters 2 nahezu kein Strömungswiderstand vorhanden ist.

Der distale Filterabschnitt 6 des Filters 2 liegt im expandierten Zustand im Bereich 15 stromaufwärts vor den Arterien zur Versorgung des Gehirns und der Arme zumindest in seinem distalen Endbereich dicht an der Gefäßwandung der aufsteigenden Aorta an. Deshalb ist es zweckmäßig die Gitterstruktur des distalen Endbereichs des distalen Filterabschnits 6 mit einer höheren Steifigkeit in radialer Richtung als den restlichen Filterabschnitt 6 auszubilden. Eine derart erhöhte Steifigkeit in diesem Bereich kann beispielsweise durch einen vergrößerten Durchmesser der Gitterstruktur, dickere Gitterstreben die dann im Katheterlumen unter erhöhter Vorspannung stehen, eine umlaufende konvexe Aufweitung in radialer Richtung, eine radial aufweitende Ausbildung oder andere geeignete Maßnahmen realisiert werden. Der restliche sich in proximaler Richtung erstreckende Abschnitt des distalen Filterabschnitts 6 bzw. dessen Gitterstruktur kann derart ausgebildet sein, dass eine hohe Steifigkeit in Längsrichtung gegeben ist.

Der distale Filterabschnitt 6 des Filters 2 kann derart röhrenförmig ausgebildet sein, dass er flächig an der Gefäßwandung des Aortabogens im Bereich der Abzweigungen zu den Arterien zur Versorgung des Gehirns und der Arme anliegen. Die Abzweigungen zu den Arterien zur Versorgung des Gehirns und der Arme sind vom Filterelement 8 des Filters 2 vollständig abgedeckt. Auf diese Weise wird in die Koronararterien abzweigendes Blut durch die kleineren Poren im distalen Filterabschnitt 6 des Filters 2 engmaschig gefiltert. Dadurch werden auch sehr kleine Verschmutzungen aus dem Blut gefiltert und können nicht ins Gehirn gelangen.

Der Durchfluss bzw. die Hauptströmung des Blutes wird durch das weitmaschigere Filterelement 8 bzw. die größeren Öffnungen im proximalen Abschnitt 7 des Filters 2 weniger intensiv gefiltert. Der proximale Abschnitt 7 des Filters 2 bildet nur einen geringen Strömungswiderstand. Der Rückstau des Blutes wird hierdurch minimal gehalten und gleichzeitig wird eine optimale Filterung des in die Koronararterien abzweigenden Blutes erzielt.

Das proximale Ende des proximalen Abschnitts 7 ist über einen Endring 9 mit einem Filterdraht 10 verbunden, der sich verschiebbar durch das Katheterlumen 5 des Katheterschlauchs 3 von distal nach proximal erstreckt. Der Endring 9 ist aus einem röntgendichten Material wie z.B. Stahl, Nitinol und Gold ausgebildet.

Der Endring 9 bzw. dessen Position kann mittels Röntgenstrahlen detektiert werden. Durch diese Detektion des Endrings 9 kann die Position des Filterkatheters 1 bzw. dessen Anordnung im Aortabogen kontrolliert werden. Weiterhin kann vorgesehen sein, dass im distalen Endbereich des distalen Filterabschnitts 6 Marker aus z.B. Gold angeordnet sind, um auch das distale Ende des Filterabschnitts 6 mittels Röntgenstrahlen zu detektieren.

Durch Verschieben des Filterdrahtes 10 innerhalb des Katheterschlauchs 3 von proximal nach distal ist der Filter 2 aus dem Katheterschlauch herausschiebbar wobei er sich aufgrund des Materials und der Ausbildung der Gitterstruktur entfaltet.

In einem zweiten Ausführungsbeispiel der vorliegenden Erfindung weist der Filter 2 eine Form analog zum ersten Ausführungsbeispiel, mit einem proximalen Abschnitt 7 und einem distalen Filterabschnitt 6, auf (Fig. 3). Der Filter 2 ist wiederum aus einer Nitinol-Gitterstruktur ausgebildet, die derartig enge Maschen aufweist, dass die Gitterstruktur das Filterelement 8 bildet. Die Größe der Öffnungen der Gitterstruktur entspricht dem oben beschriebenen Ausführungsbeispiel, wobei die Gitterstruktur ebenfalls mit einer antitrombogenen Beschichtung beschichtet sein kann.

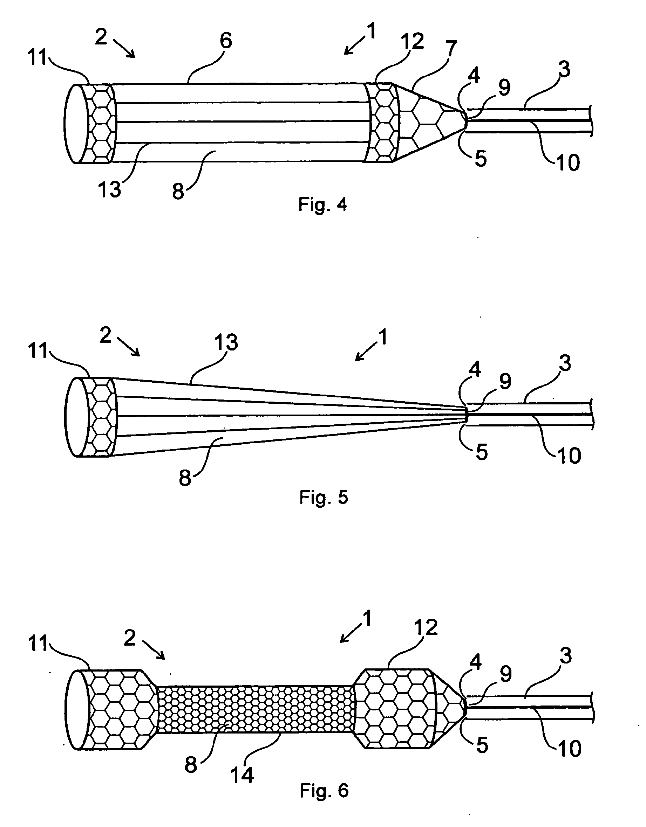

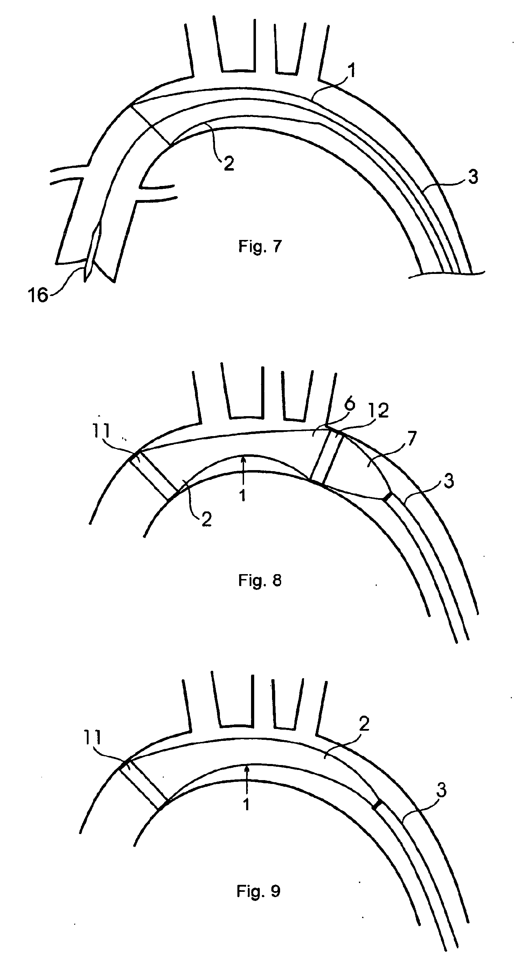

In einem dritten Ausführungsbeispiel der vorliegenden Erfindung ist ebenfalls ein distaler Filterabschnitt 6 und ein proximaler Abschnitt 7 vorgesehen (Fig. 4, 8). Am distalen und am proximalen Ende des distalen Filterabschnitts 6 ist jeweils ein distaler Ringabschnitt 11 und ein proximaler Ringabschnitt 12 ausgebildet. Die ringförmigen Abschnitte 11, 12 sind aus Nitinol ausgebildet. Die beiden ringförmigen Abschnitte 11, 12 sind über sich in Längsrichtung erstreckende Streben 13 miteinander verbunden.

Die Ringabschnitte 11, 12 sind im vorliegenden Ausführungsbeispiel aus einer ringförmigen Gitterstruktur ausgebildet, die die notwendige Festigkeit zum dichten Anliegen an der Blutgefäßwandung besitzt.

Es kann auch vorgesehen sein, dass die Gitterstrukturen der Ringabschnitte 11, 12 einen größeren Durchmesser als der Abschnitt 7 oder eine umlaufende konvexe Aufweitung in radialer Richtung aufweisen bzw., dass die Ringabschnitte 11, 12 jeweils an ihrem distalen bzw. proximalen Endbereich radial aufweitend ausgebildet sind.

Die Ringabschnitte 11, 12 sind aus einer Formgedächtnislegierung ausgebildet und mit einem Filterelement 8 versehen.

Der distale Ringabschnitt und der proximale Ringabschnitt 11, 12 weisen eine Länge von in etwa 0,1 cm bis 3 cm auf.

Die Streben bzw. drahtförmigen Abstandshalter 13 sind in etwa gleichmäßig voneinander beabstandet. Entlang des Umfangs des distalen und des proximalen Ringabschnitts 11, 12 sind in etwa 4 bis 10 der Streben 13 vorgesehen. Die Streben 13 sind aus einer Formgedächtnislegierung, wie z.B. Nitinol ausgebildet, und weisen eine Länge von in etwa 8 cm bis 14 cm auf.

Auf der Innenseite der Streben 13 und auf der Innenseite der Gitterstruktur des proximalen Abschnitts 7 ist eine Kunststofffolie als Filterelement 8 angeordnet. Die Kunststofffolie weist im Bereich der Streben 13 Filteröffnungen mit einem Durchmesser von in etwa 100 µm bis 200 µm auf. Im proximalen Abschnitt 7 sind Filteröffnungen mit einem Durchmesser von in etwa 200 µm bis 300 µm vorgesehen. Aus oben bereits genannten Gründen ist vorgesehen, dass die Filteröffnungen im Bereich der Streben 13 einen geringeren Durchmesser aufweisen als die Filteröffnungen im proximalen konischen Abschnitt 7.

Am proximalen Ende des proximalen Abschnitts 7 ist ein röntgendichter Endring 9 angeordnet der mit dem Filterdraht 10 verbunden ist.

Weiterhin kann vorgesehen sein, den distalen und/oder den proximalen Ringabschnitt 11, 12 aus einer Gitterstruktur, die als Filterelement 8 dient, gemäß dem zweiten Ausführungsbeispiel auszubilden.

Bei beiden eben beschriebenen Ausführungsformen ist vorgesehen, die Filteröffnungen der Kunststofffolie im distalen Filterabschnitt 6 kleiner als die Filteröffnungen im proximalen Abschnitt 7 auszubilden.

Es kann auch vorgesehen sein, dass der proximale Abschnitt 7 aus sich in Längsrichtung erstreckenden Streben oder einer Gitterstruktur mit großen Öffnungen ausgebildet ist.

In einem vierten Ausführungsbeispiel der vorliegenden Erfindung weist der Filterkatheter 1 einen langgestreckten trichterförmigen bzw. sich von distal nach proximal verjüngenden Filter 2 auf (Fig. 5, 8).

Der Filter weist eine Länge von in etwa 5 cm bis 16 cm bzw. eine Länge von in etwa 6 cm bis 15 cm, bzw. eine Länge von in etwa 5 cm bis 14 cm, bzw. eine Länge von in etwa 6 cm bis 14 cm, bzw. eine Länge von in etwa 7 cm bis 14 cm, bzw. eine Länge von in etwa 8 cm bis 14 cm und vorzugsweise eine Länge von in etwa 10 cm bis 14 cm und einen Durchmesser im expandierten Zustand im distalen Endabschnitt von in etwa 3 cm bis 6 cm auf.

Am distalen Ende des Filters 2 ist ein Ringabschnitt 11 aus Nitinol ausgebildet. Es kann auch vorgesehen sein, den Ringabschnitt 11 gemäß dem oben beschriebenen Ausführungsbeispiel auszubilden. Der Ringabschnitt 11 weist eine Länge von in etwa 0,1 cm bis 3 cm auf. Der Ringabschnitt 11 kann wie im vorigen Ausführungsbeispiel beschrieben ausgebildet sein.

Entlang des Umfangs des ringförmigen Abschnitts 11 sind sich in longitudinaler proximaler Richtung erstreckende dünne stabförmige Streben 13 angeordnet. Es sind in etwa 4 bis 10 Streben 13 vorgesehen, die in etwa gleichmäßig entlang des Umfangs des Ringabschnitts 11 angeordnet sind.

Die Streben 13 bilden von distal nach proximal einen sich verjüngenden Konus bzw. Kegel aus, an dessen proximalem Ende ein röntgendichter Endring 9 angeordnet ist, der alle Streben 13 fixiert und mit dem Filterdraht 10 verbindet.

An der Innenseite der Streben 13 ist als Filterelement 8 eine Kunststofffolie angeordnet, deren Poren einen Durchmesser von in etwa 100 µm bis 300 µm aufweisen.

Die Größe der Filteröffnungen ist derart gewählt, dass das Blut von kleinen Verschmutzungen gereinigt wird. Da zur Reinigung des Blutes eine große Filterfläche zur Verfügung steht und die Reinigung über die gesamte Filterfläche erfolgt, wird die Strömungsgeschwindigkeit des Blutes nur geringfügig beeinflusst und nur ein geringer Gegendruck aufgebaut, selbst wenn die Größe der Filteröffnungen im wesentlichen konstant ist. Bevorzugt wird jedoch eine Ausführungsform mit einem distalen Filterabschnitt 6 und einem proximalen Abschnitt 7, wobei im proximalen Abschnitt größere Öffnungen als im distalen Filterabschnitt 6 ausgebildet sind. Der distale Filterabschnitt 6 erstreckt sich über die Hälfte bis zu Zwei-Drittel der Länge des Filters 2.

Der Filter 2 bzw. das darin angeordnete Filterelement 8 kann von distal nach proximal in mehrere Bereiche, beispielsweise drei Bereiche unterteilt sein. Das Filterelement 8 der drei Bereiche weist dann von distal nach proximal immer größer werdende Filteröffnungen auf. Die Filteröffnungen des ersten Bereichs weisen einen Durchmesser von in etwa 100 µm bis 200 µm, die des zweiten einen Durchmesser von in etwa 150 µm bis 300 µm und die des dritten Bereichs einen Durchmesser von in etwa 200 µm bis 400 µm auf.

Weiterhin kann auch vorgesehen sein, auf ein Filterelement 8 im proximalen Abschnitt 7 zu verzichten, um den Durchfluss des Blutes nicht zu behindern.

Das Filterelement 8 kann aus einem der bereits oben beschriebenen Materialien, wie z.B. PU, PTFE, PP PE, ausgebildet sein.

Durch die unterschiedlich großen Filteröffnungen wird sichergestellt, dass das in die Arterien zur Versorgung des Gehirns und der Arme fließende Blut ausreichend fein gefiltert wird und die Hauptströmung des Blutes im proximalen Bereich des Filters 2 durch die größeren Filteröffnungen des Filterelements 8 bzw. die Öffnungen der Gitterstruktur in diesem Bereich nur geringfügig beeinflusst wird.

Es kann auch vorgesehen sein, den Filter 2 anstelle der Streben aus einer Gitterstruktur aus einer Formgedächtnislegierung auszubilden, auf deren Innenseite eine Kunststofffolie als Filterelement 8 angeordnet ist. Weiterhin kann die Gitterstruktur aus einer Formgedächtnislegierung das Filterelement 8 bilden und somit auf die Kunststofffolie verzichtet werden kann.

Wenn keine Kunststofffolie vorgesehen wird, dann kann der Filter 2 mit einer antitrombogenen Beschichtung versehen sein.

Der langgestreckte sich verjüngende Filter 2 weist eine hohe Steifigkeit in Längsrichtung auf, um ein zu starkes Abknicken des Filters im Aortabogen zu verhindern. Die Steifgkeit kann z.B. durch dickere Streben oder durch dickere Streben der Gitterstruktur oder durch die Anordnung der Streben der Gitterstruktur realisiert werden.

Der Filter 2 kann auch ohne den ringförmigen Abschnitt 11 ausgebildet sein, beispielsweise bei Verwendung einer Gitterstruktur als Filter 2, wobei der distale Enbereich der Gitterstruktur derart ausgebildet ist bzw. die notwendige Steifigkeit aufweist, dass er dicht mit der Gefäßwandung abschließt.

In einem fünften Ausführungsbeispiel der vorliegenden Erfindung ist ein Filter 2 in etwa hantelförmig ausgebildet (Fig. 6). Am distalen und am proximalen Ende des Filters 2 ist jeweils ein Ringabschnitt 11, 12 ausgebildet. Die Ringabschnitte 11, 12 weisen eine Gitterstruktur und eine Länge von in etwa 0,5 cm bis 5 cm auf. Die Gitterstrukturen des distalen und des proximalen Ringabschnitts 11, 12 weisen die notwendige Festigkeit zum dichten Anliegen an der Gefäßwandung des Blutgefäßes auf.

Im Bereich zwischen den beiden Ringabschnitten 11, 12 ist ein röhrenförmiger Filterabschnitt 14 vorgesehen. Dieser ist in etwa 3 cm bis 12 cm lang und weist aufgrund der Ausbildung der Gitterstruktur nur eine geringe Steifigkeit in radialer Richtung und eine hohe Festigkeit in Längsrichtung auf. Dieser röhrenförmig ausgebildete Filterabschnitt 14 weist einen geringeren Durchmesser als die beiden Ringabschnitte 11, 12 auf.

Am proximalen Ende des Ringabschnitts 12 des Filters 2 ist ein sich verjüngender proximaler Abschnitt 7 vorgesehen, an dessen Ende ein röntgendichter Endring 9 angeordnet ist.

Auf der Innenseite der Gitterstruktur des röhrenförmigen Filterabschnitts 14 ist eine Kunststofffolie angeordnet, deren Filteröffnungen einen Durchmesser von in etwa 110 µm bis 300 µm aufweisen.

Auf der Innenseite der Gitterstruktur des proximalen Abschnitts 7 ist eine Kunststofffolie angeordnet, deren Filteröffnungen größer als die Filteröffnungen des röhrenförmigen Filterabschnitts sind und z.B. einen Durchmesser von in etwa 200 µm bis 400 µm aufweisen.

In einem weiteren Ausführungsbeispiel der vorliegenden Erfindung weist der Filter eine Form analog zum fünften Ausführungsbeispiel, mit einem hantelförmig ausgebildten Filter 2 auf. Dabei bildet die Gitterstruktur des Filters 2 das Filterelement 8 aus. Die Größe der Filteröffnungen der Gitterstruktur entspricht dem fünften Ausführungsbeispiel.

Bei allen Ausführungsbeispielen können Marker aus einem röntgendichten Material am distalen Ende des Filters vorgesehen sein.

Alle oben beschriebenen Ausführungsbeispiele können auch mit Filteröffnungen ausgebildet sein, die im wesentlichen über die gesamte Länge - also im distalen Filterabschnitt 6 als auch im proximalen Abschnitt 7 - des Filters eine etwa konstante Größe mit einem Durchmesser im Bereich von etwa 150 µm bis 250 µm und vorzugsweise 150 µm bis 200 µm aufweisen. Diese Öffnungsgröße ist sowohl ausreichend klein um Partikel aus dem zum Gehirn strömenden Blut zu filtern als auch noch groß genug um den Rückstau gering zu halten. Dies gilt insbesondere für die Ausführungsform des Filters, der sich allmählich von distal nach proximal verjüngt, da hier eine sehr große Filterfläche vorgesehen wird. Eine in Richtung zum proximalen Ende des Filters zunehmende Öffnungsgröße wird jedoch bevorzugt.

Bei den oben beschriebenen Ausführungsbeispielen ist jeweils ein Filterdraht zum Ausschieben des Filters aus dem Katheter vorgesehen. Anstelle des einen Filterdrahtes können auch mehrere Filterdrähte oder ein schlauchförmiger Innenkatheter, der mit dem Filter fest verbunden ist und bzgl. eines schlauchförmigen Außenkatheters beweglich ausgebildet ist, vorgesehen sein.

Weiterhin ist der Filterkatheter vorzugsweise derart ausgebildet, dass im ausgefahrenen Zustand des Filters dieser an dem Katheterlumen mündet, d.h., dass der Katherschlauch nicht in den Filter vorsteht.

Bei obigen Ausführungsbeispielen ist der Filter jeweils selbstexpandierend ausgebildet. Im Rahmen der Erfindung ist es auch möglich die Expansion durch einen Ballon zu unterstützen oder alleine durch einen Ballon auszuführen. Jedoch sind hier die Möglichkeiten in der Formgestaltung des Filters stark begrenzt, weshalb ein selbstexpandierender Filter bevorzugt wird.

Im folgenden wird das Verfahren zum Filtern von Blut erläutert. Hierbei können alle oben beschriebenen Ausführungsbeispiele verwendet werden.

Wie in Fig. 1 dargestellt, wird der Katheter über die Femoral-Arterie oder die Radialis-Arterie oder die Subklavias-Arterie, über die absteigende Aorta in den Aortabogen eingeführt. Der Katheter wird somit entgegen der Strömungsrichtung des Blutes eingeführt und im Aortabogen im Bereich der Abzweigungen der Arterien zur Versorgung des Gehirns und der Arme angeordnet.

Wenn der Filterkatheter 1 korrekt positioniert ist, wird der Filter 2 aus dem Katheterlumen 5 des Katheterschlauchs 3 durch Bewegung des Filterdrahtes 10 von proximal nach distal ausgeschoben.

Die Positionierung des Filters 2 kann durch Detektion der Marker am distalen Ende des Filters 2 oder des röntgendichten Endrings 9 kontrolliert werden.

Bei Verwendung eines Filterkatheters 1 mit einem röhrenförmigen Filter 2 expandiert sich der Filter 2 radial derart, dass zumindest sein distaler Endbereich an der Gefäßwandung und vorzugsweise der gesamte distale Filterabschnitt 6 an der Gefäßwandung der aufsteigenden Aorta und insbesondere im Bereich der Abzweigungen zu den Arterien zur Versorgung des Gehirns und der Arme anliegt.

Bei Verwendung eines Filterkatheters 1 mit einem trichterförmigen Filter 2 expandiert sich der Filter 2 radial derart, dass sein distaler Endbereich an der Gefäßwandung der aufsteigenden Aorta im Bereich vor den Abzweigungen zu den Arterien zur Versorgung des Gehirns und der Arme anliegt und mit der Gefäßwandung dicht abschließt.

Bei Verwendung eines Filterkatheters 1 mit einem hantelförmigen Filter 2 expandiert sich der Filter 2 radial derart, dass zumindest sein distaler Endbereich bzw. der distale Ringabschnitt 11 an der Gefäßwandung und vorzugsweise der distale und der proximale Ringabschnitt 11, 12 des Filters entsprechend an der Gefäßwandung des Aortabogens im Bereich vor und hinter den Abzweigungen zu den Arterien zur Versorgung des Gehirns und der Arme anliegen. Um den Anpressdruck an die Gefäßwandung zu erhöhen weisen der distale und der proximale Ringabschnitt 11, 12 des Filters 2 eine hohe Steifigkeit in radialer Richtung auf.

Auf diese Weise ist es möglich, das von distal nach proximal durch den Filter 2 strömende Blut von embolischem Material, Plaque und Debris zu reinigen. Insbesondere ist dabei vorgesehen, das in die Arterien zur Versorgung des Gehirns und der Arme abzweigende Blut von besonders kleinen Partikeln zu reinigen, um die Gefahr eines Hirnschlags zu minimieren.

Durch die bereits beschriebene gröbere Ausbildung des Filters 2 im proximalen Bereich des Filters 2 bzw. das vollständige Weglassen eines Filterelements im proximalen Bereich des Filters 2 wird die Hauptströmung des Blutes nur geringfügig oder gar nicht beeinflusst und somit nur ein geringer Gegendruck aufgebaut. Das Blut kann ungehindert oder nur mit geringem Rückstau weiter fließen.

Der Filter 2 wird durch Bewegung des Filterdrahtes 10 von distal nach proximal wieder in das Katheterlumen 5 des Katheterschlauchs 3 eingezogen. Der Filter 2 kann beim Einziehen in das Katheterlumen unbeschädigt bleiben. Dies muss aber nicht notwendigerweise so sein. Das aus dem Blut gefilterte embolische Material, Plaque und Debris verbleiben beim Einziehen des Filters 2 vollständig im Filter 2 und werden mit in den Katheterschlauch 3 eingezogen.

Die Vorrichtung kann selbstverständlich auch über jeden anderen bekannten Zugang zugeführt werden und nicht ausschließlich nur über die Femoral-Aorta.

Bei der erfindungsgemäßen Vorrichtung ist von Vorteil, dass durch das Vorsehen eines Filters 2 am distalen Ende des hohlen Katheterschlauchs 3, wobei der Filter 2 ein selbstentfaltendes Tragwerk aus einem Formgedächtnismaterial umfasst, eine einfache und schnelle Anordnung des Filters 2 möglich ist.

Das Katheterlumen 5 des Katheterschlauchs 3 weist vorzugsweise über seine gesamte Länge einen konstanten Innendurchmesser auf. Es kann zweckmäßig sein, den Innendurchmesser des Katheterschlauchs 3 im distalen Endbereich etwas größer als im übrigen Bereich des Katheterschlauchs 3 zu gestalten, damit hier ausreichend Platz zur Aufnahme des Filters 2 vorhanden ist. Der übrige Bereich des Filterkatheters 1 kann nicht beliebig dick ausgebildet werden, da er zum Einführen in die gebogene Aorta eine gewisse Flexibilität besitzen muss, die bei einem Durchmesser von deutlich mehr als 21 French (7 mm) bei üblicherweise verwendeten Kathetermaterialien nicht immer gegeben ist.

Alle oben beschriebenen Ausführungsbeispiele können derart ausgebildet sein, dass der Filterkatheter ein Katheterlumen aufweist, dass z.B. ein Ballonkatheter 16 hindurchgeführt werden kann. Das Katheterlumen des Katheterschlauchs weist einen Durchmesser von ca. 4 mm bis 7 mm auf, so dass der Ballonkatheter hindurch geführt werden kann. Ein solch großes Katheterlumen erlaubt den Einsatz eines Ballonkatheters mit dem eine Stenose der Herzklappe beseitigt.

Mit dem Ballonkatheter wird zunächst ein Dilatationsballon im Bereich der Herzklappe gesetzt. Der Dilatationsballon wird mittels eines Führungsdrahtes durch die Herzklappe geführt. Anschließend wird der Ballon aufgeblasen, wodurch die natürliche Herzklappe weggesprengt wird.

Beim Sprengen der natürlichen Herzklappe mittels des Ballonkatheters wird Belag (Plaque bzw. Debris) gelöst. Das während des Sprengens der Herzklappe passierende Blut wird über den Filter gefiltert, der an dem stromabwärts der Herzklappe angeordneten Katheterschlauch ausgebildet ist.

Die Herzklappe kann mit dem Ballonkatheter derart stark aufgeweitet werden, dass eine künstliche Herzklappe eingesetzt werden kann. Diese künstliche Herzklappe kann mittels eines weiteren Katheters durch das Lumen des Filterkatheters zugeführt und platziert werden. Eine künstliche Aortaklappe ist z. B. aus der EP 1 335 683 B1 bekannt. Weitere implantierbare Herzklappenprothesen sowie Katheter für die Implantation einer solchen Herzklappenprothese gehen aus der EP 592 410 B1, der US 2004/0210304 A1, US 7,018,406 B2, W02006/127765 A1, der US 2003/0036795 A1, der US 5,411,552, der US 6,168,614 B1, der US 6,582,462 B1 und der WO91/17720 hervor. Auf diese Dokumente wird hiermit vollinhaltlich Bezug genommen.

Das Blut kann auch durch das Katheterlumen des Katheterschlauchs nach außen fließen und durch eine Herz-Lungen-Maschine oder eine andere externe Filtereinrichtung geleitet werden.

Der Katheterschlauch weist an seinem zum Bedienen des Filters benachbarten Ende, dem proximalen Ende, ein Einwegventil (hemostatic valve) auf, durch das der den Ballon setzende Ballonkatheter hindurchgeführt werden kann. Beim Entfernen des Ballonkatheters schließt das Ventil automatisch, so dass ein Herauslaufen von Blut verhindert wird.

Weiterhin kann vorgesehen sein, dass das im inneren des Filters angesammelte Material, wie z.B. Plaque, Debris oder eine Embolie auslösendes Material, durch Anlegen eines Unterdrucks am proximalen Ende des Katheterschlauchs über das Katheterlumen abgesaugt wird.

Bei einer weiteren vorteilhaften Ausführungsform wird ein femoraler Beipass gelegt, wobei am Beipass eine Herz-Lungen-Maschine angeordnet ist, die das Blut filtert. Eine solche erfindungsgemäße Vorrichtung kann auch dann von Vorteil sein, wenn so viel Plaque und Debris anfallen, dass eine externe Filterung notwendig wird.

Beim Setzen eines femoralen Beipasses wird ein Katheterschlauch verwendet, der an seinem proximalen Ende eine Y-Verzweigung aufweist, wobei ein Ende der Y-Verzweigung mit dem Einwegventil versehen ist und das andere eine Kappe aufweist, die zum Anschließen der das Blut filternden Herz-Lungen-Maschine gelöst werden kann. Im Gegensatz zum Stand der Technik wird der Filter erfindungsgemäß stromabwärts einer aufzusprengenden Herzklappe angeordnet, wobei der langgestreckte Filter sich im Gegensatz zum Stand der Technik über den Aortabogen und insbesondere über den Bereich, in dem die Arterien zur Versorgung des Gehirns und der Arme angeordnet sind, erstreckt.

Die Erfindung kann folgendermaßen kurz zusammengefasst werden:

Die Vorrichtung zum Filtern von Blut umfasst einen Filterkatheter welcher zum Filtern des stromabwärts hinter der Herzklappe in die Arterien zur Versorgung des Gehirns und der Arme abzweigenden Blutes an seinem distalen Ende einen Filter aufweist, welcher aus einem Katheterschlauch distal ausschiebbar ist. Der Filter ist röhrenförmig oder sich von distal nach proximal verjüngend ausgebildet. Der Filter ist derart ausgebildet, dass er im Bereich stromaufwärts vor den Arterien zur Versorgung des Gehirns und der Arme zumindest an seinem distalen Endbereich dicht an der aufsteigenden Aorta anliegt.

### Bezugszeichenliste

- 1: Filterkatheter
- 2: Filter
- 3: Katheterschlauch
- 4: distales Ende
- 5: Katheterlumen
- 6: distaler Filterabschnitt
- 7: proximaler Abschnitt
- 8: Filterelement
- 9: Endring
- 10: Filterdraht
- 11: distaler Ringabschnitt
- 12: proximaler Ringabschnitt
- 13: Streben
- 14: röhrenförmiger Abschnitt
- 15: Bereich
- 16: Ballonkatheter

## Patentansprüche

1. Vorrichtung zum Filtern von Blut, umfassend einen Filterkatheter (1) welcher zum Filtern des stromabwärts hinter der Herzklappe in die Arterien zur Versorgung des Gehirns und der Arme abzweigenden Blutes an seinem distalen Ende einen Filter (2) aufweist, welcher aus eine Gitterstruktur ausgebildet ist und aus einem Katheterschlauch (3) distal ausschiebbar ist, und der Filter (2) im ausgeschobenen Zustand sich in Richtung von proximal nach distal aufweitend ausgebildet ist, wobei der Filter einen distalen Filterabschnitt (6) und einen proximalen Abschnitt (7) aufweist, und der distale Filterabschnitt (6) Filteröffnungen aufweist und der proximale Abschnitt (7) eine oder mehrere Öffnungen aufweist, die größer als die Filteröffnungen des distalen Filterabschnitts (6) ausgebildet sind, und die Gitterstruktur einen distalen Endbereich zum dichten Anliegen an der aufsteigenden Aorta aufweist, **dadurch gekennzeichnet, dass** zumindest der distale Endbereich des distalen Filterabschnitts (6) mit einer höheren Steifigkeit in radialer Richtung als der restliche Filterabschnitt ausgebildet ist.

2. Vorrichtung nach Anspruch 1,
**dadurch gekennzeichnet,**
**dass** der Filter (2) länger als ein Abschnitt im Aortabogen ist, in dem die Arterien zur Versorgung des Gehirns und der Arme abzweigen, der im folgenden als Aortabogenabschnitt bezeichnet wird, und der distale Filterabschnitt (6) röhrenförmig ist und der proximale Abschnitt (7) sich vom distalen Filterabschnitt (6) zum Katheterschlauch (3) verjüngt, und dass der Filter zumindest im Bereich stromaufwärts vor den Arterien zur Versorgung des Gehirns und der Arme an seinem distalen Endbereich dicht an der aufsteigenden Aorta anliegend ausgebildet ist.

3. Vorrichtung nach Anspruch 1,
**dadurch gekennzeichnet,**
**dass** der langgestreckte Filter(2) sich von distal nach proximal verjüngenden ausgebildet ist, und der Filter (2) länger als ein Abschnitt im Aortabogen ist, in dem die Arterien zur Versorgung des Gehirns und der Arme abzweigen, der im folgenden als Aortabogenabschnitt bezeichnet wird, und der Filter (2) derart ausgebildet ist, dass er im Bereich stromaufwärts vor diesen Arterien zumindest mit seinem distalen Endbereich dicht an der aufsteigenden Aorta anlegbar ist.

4. Vorrichtung nach einem der Ansprüche 1 bis 3,
**dadurch gekennzeichnet,**
**dass** der Filter (2) eine Länge von 5 cm bis 18 cm aufweist, insbesondere eine Länge von 8 cm bis 16 cm bzw. eine Länge von 10 cm bis 14 cm aufweist.

5. Vorrichtung nach einem der Ansprüche 1 bis 4,
**dadurch gekennzeichnet,**
**dass** der Filter (2) eine Gitterstruktur aus einem Formgedächtnismaterial aufweist.

6. Vorrichtung nach Anspruch 5,
**dadurch gekennzeichnet,**
**dass** auf der Innenseite oder der Außenseite der Gitterstruktur ein aus einer Kunststofffolie ausgebildetes Filterelement (8) angeordnet ist.

7. Vorrichtung nach einem der Ansprüche 1 bis 6,
**dadurch gekennzeichnet,**
**dass** die Filteröffnungen im Filterabschnitt (6) einen Durchmesser von in etwa 100 µm bis 400 µm aufweisen.

8. Vorrichtung nach einem der Ansprüche 1 bis 7,
**dadurch gekennzeichnet,**
**dass** die Filteröffnungen von distal nach proximale größer werden.

9. Vorrichtung nach einem der Ansprüche 1 bis 8,
**dadurch gekennzeichnet,**
**dass** der Filterkatheter (1) ein Katheterlumen aufweist, das derart ausgebildet ist, dass ein weiterer Katheter durch das Katheterlumen hindurch geführt werden kann, wobei das Katheterlumen vorzugsweise einen Durchmesser von ca. 4 mm bis 7 mm aufweist.

10. Vorrichtung nach einem der Ansprüche 1 bis 9,
**dadurch gekennzeichnet,**
**dass** der Katheterschlauch (3) am distalen Endbereich, in dem der Filter (2) aufgenommen werden kann mit einem größeren Umfang als an seinem übrigen Bereich
ausgebildet ist, so dass der Durchmesser des Katheterlumens über die gesamte Länge des Katheterschlauchs (3) im wesentlichen konstant ist.

11. Vorrichtung nach einem der Ansprüche 1 bis 10,
**dadurch gekennzeichnet,**
**dass** das proximale Ende des Katheterschlauchs (3) zum Anschließen an eine Herz-Lungen-Maschine und/oder eines externen Filters ausgebildet ist.

12. Vorrichtung nach einem der Ansprüche 1 bis 11,
**dadurch gekennzeichnet,**
**dass** im Katheterlumen (5) des Katheterschlauchs (3), vorzugsweise am proximalen Ende des Filterkatheters, ein Einweg-Ventil angeordnet ist, durch das ein weiterer Katheter hindurch geführt werden kann.

13. Vorrichtung nach einem der Ansprüche 9 bis 12,
**dadurch gekennzeichnet,**
**dass** der weitere Katheter ein Ballonkatheter (16) ist, der durch das Katheterlumen (5) des Katheterschlauchs (3) vom proximalen zum distalen Ende hindurch geführt werden kann, wobei der Ballonkatheter (16) an seinem distalen Ende mit einem Ballon zur Beseitigung einer Herzklappenstenose versehen ist.

14. Vorrichtung nach einem der Ansprüche 1 bis 13, **dadurch gekennzeichnet,**
**dass** der Filter (2) langgestreckt ausgebildet istund an seinem proximalen Ende in einen Katheterschlauch (3) übergeht, wobei der Filter (2) aus dem Katheter distal ausschiebbar ist, und der Filter (2) länger als ein Abschnitt im Aortabogen ist, in dem die Arterien zur Versorgung des Gehirns und der Arme abzweigen, der im folgenden als Aortabogenabschnitt bezeichnet wird, und der Filter (2) derart ausgebildet ist, dass er im Bereich stromaufwärts vor diesen Arterien zumindest mit seinem distalen Endbereich dicht an der aufsteigenden Aorta anliegt, wobei der Filter entweder sich von distal nach proximal verjüngenden ausgebildet ist, oder der Filter einen distalen röhrenförmigen Filterabschnitt (6) und einen proximalen Abschnitt (7) aufweist, der sich vom distalen Filterabschnitt (6) zum Katheterschlauch (3) verjüngt, ausgebildet ist.

15. Vorrichtung nach Anspruch 14,
**dadurch gekennzeichnet,**
**dass** der Filter (2) Filteröffnungen mit einem Durchmesser von etwa 150 bis 250 µm aufweist.

## Claims

1. Device for the filtering of blood, comprising a filter catheter (1) with a filter (2) at its distal end for filtering the blood branching downstream behind the heart valve into the arteries to supply the brain and the arms, and which is made of a lattice structure, wherein the filter (2) may be pushed out of a catheter tube (3) in the distal direction and wherein the filter (2) is designed to expand from the proximal to the distal in the extended state, and wherein the filter has a distal filter section (6) and a proximal section (7), and the distal filter section (6) has filter openings, and the proximal section (7) has one or more openings which are larger than the filter openings of the distal filter section (6), and the lattice structure has a distal end section to fit up tightly against the ascending aorta, **characterised in that** at least the distal end section of the distal filter section (6) is designed with greater stiffness in the radial direction as the rest of the filter section.

2. Device according to claim 1,
**characterised in that**
the filter (2) is longer than a section in the aortic arch in which the arteries which supply the brain and the arms branch off, subsequently described as the aortic arch section, and the distal filter section (6) is tubular and the proximal section (7) tapers from the distal filter section (6) to the catheter tube (3), and that the filter is designed to fit tightly with its distal end section against the ascending aorta, at least in the area upstream of the arteries which supply the brain and the arms.

3. Device according to claim 1,
**characterised in that**
the elongated filter (2) is designed to taper from the distal to the proximal end, and the filter (2) is longer than a section in the aortic arch in which the arteries which supply the brain and the arms branch off, subsequently described as the aortic arch section, and that the filter (2) is designed so as to be capable of fitting tightly with its distal end section against the ascending aorta, at least in the area upstream of these arteries.

4. Device according to any of claims 1 to 3,
**characterised in that**
the filter (2) has a length of 5 cm to 18 cm, in particular a length of 8 cm to 16 cm or a length of 10 cm to 14 cm.

5. Device according to any of claims 1 to 4,
**characterised in that**
**that** the filter (2) has a lattice structure made of memory material.

6. Device according to claim 5,
**characterised in that**
a filter element (8) made of plastic film is provided on the inside or the outside of the lattice structure.

7. Device according to any of claims 1 to 6,
**characterised in that**
the filter openings in the filter section (6) have a diameter of around 100 µm to 400 µm.

8. Device according to any of claims 1 to 7,
**characterised in that**
the filter openings increase in size from the distal to the proximal end.

9. Device according to any of claims 1 to 8,
**characterised in that**
the filter catheter (1) has a catheter lumen which is so designed that another catheter may be guided through the catheter lumen, wherein the catheter lumen preferably has a diameter of around 4 mm to 7 mm.

10. Device according to any of claims 1 to 9,
**characterised in that**
the catheter tube (3) is provided with a greater circumference at the distal end section in which the filter (2) may be accommodated than in its other sections, so that the diameter of the catheter lumen is substantially constant over the entire length of the catheter tube (3).

11. Device according to any of claims 1 to 10,
**characterised in that**
the proximal end of the catheter tube (3) is designed for connection to a heart-lung machine and/or an external filter.

12. Device according to any of claims 1 to 11
**characterised in that**
a one-way valve through which another catheter may be guided is provided in the catheter lumen (5) of the catheter tube (3), preferably at the proximal end of the filter catheter.

13. Device according to any of claims 9 to 12,
**characterised in that**
the other catheter is a balloon catheter (16), which may be guided through the catheter lumen (5) of the catheter tube (3) from the proximal to the distal end, wherein the balloon catheter (16) is provided at its distal end with a balloon for the removal of a heart valve stenosis.

14. Device according to any of claims 1 to 13,
**characterised in that**
the filter (2) is elongated and merges at its proximal end into a catheter tube (3), wherein the filter (2) may be pushed out of the catheter in the distal direction, and the filter (2) is longer than a section in the aortic arch in which the arteries which supply the brain and the arms branch off, subsequently described as the aortic arch section, and the filter (2) is so designed that in the area upstream of these arteries it fits tightly against the ascending aorta, at least with its distal end section, wherein the filter either tapers from the distal to the proximal end or the filter has a distal tubular filter section (6) and a proximal section (7) which tapers from the distal filter section (6) to the catheter tube (3).

15. Device according to claim 14,
**characterised in that**
the filter (2) has filter openings with a diameter of around 150 to 250 µm.

## Revendications

1. Dispositif pour filtrer le sang, comprenant un cathéter-filtre (1) qui, pour filtrer le sang dérivé en aval de la valvule cardiaque vers les artères destinées à alimenter le cerveau et les bras, comprend à son extrémité distale un filtre (2) qui est formé par une structure grillagée et qui est susceptible d'être déployé de manière distale hors d'un tube de cathéter (3), et dans la situation déployée le filtre (2) est réalisé en s'élargissant de la direction proximale vers distale, ledit filtre comprenant un tronçon de filtre distal (6) et un tronçon proximal (7), tels que le tronçon de filtre distal (6) comporte des ouvertures de filtre et que le tronçon proximal (7) comporte une ou plusieurs ouvertures qui sont réalisées plus grandes que les ouvertures de filtre du tronçon de filtre distal (6), et la structure grillagée comprend une zone terminale distale destinée à s'appliquer de façon étanche contre l'aorte montante, **caractérisé en ce que** la zone terminale distale du tronçon de filtre distal (6) au moins est réalisée avec une rigidité supérieure en direction radiale que le tronçon de filtre restant.

2. Dispositif selon la revendication 1,
**caractérisé en ce que**
le filtre (2) est plus long qu'un tronçon dans la crosse de l'aorte dans lequel les artères destinées à l'alimentation du cerveau et des bras sont ramifiées, désigné dans ce qui suit par "tronçon de crosse d'aorte", et le tronçon de filtre distal (6) est en forme de tube et le tronçon proximal (7) va en se rétrécissant depuis le tronçon de filtre distal (6) vers le tuyau de cathéter (3), et **en ce que** le filtre est réalisé, au moins dans la région en amont des artères destinées à l'alimentation du cerveau et des bras, de manière à s'appliquer de façon étanche dans sa zone terminale distale contre l'aorte montante.

3. Dispositif selon la revendication 1,
**caractérisé en ce que**
le filtre allongé (2) est réalisé en se rétrécissant de la position distale à la position proximale, et le filtre (2) est plus long qu'un tronçon dans la crosse de l'aorte dans lequel les artères destinées à l'alimentation du cerveau et des bras sont ramifiées, désigné dans ce qui suit par "tronçon de crosse d'aorte", et le filtre (2) est réalisé de telle façon que, dans la zone en amont de ces artères, il est susceptible d'être appliqué au moins avec sa zone terminale distale de façon étanche contre l'aorte montante.

4. Dispositif selon l'une des revendications 1 à 3,
**caractérisé en ce que**
le filtre (2) présente une longueur de 5 cm à 18 cm, en particulier une longueur de 8 cm à 16 cm, ou encore une longueur de 10 cm à 14 cm.

5. Dispositif selon l'une des revendications 1 à 4,
**caractérisé en ce que** le filtre (2) présente une structure grillagée formée d'un matériau à mémoire de forme.

6. Dispositif selon la revendication 5,
**caractérisé en ce que**
un élément de filtre (8) réalisé à partir d'un film de matière plastique est agencé sur la face intérieure ou sur la face extérieure de la structure grillagée.

7. Dispositif selon l'une des revendications 1 à 6,
**caractérisé en ce que**
les ouvertures de filtre dans le tronçon de filtre (6) présentent un diamètre d'environ 100 µm à 400 µm.

8. Dispositif selon l'une des revendications 1 à 7,
**caractérisé en ce que** les ouvertures de filtre deviennent plus grandes de la position distale à la position proximale.

9. Dispositif selon l'une des revendications 1 à 8,
**caractérisé en ce que** le cathéter-filtre (1) présente un lumen de cathéter qui est réalisé de telle façon qu'il est possible de faire passer un autre cathéter à travers le lumen de cathéter, ledit lumen de cathéter présentant de préférence un diamètre d'environ 4 mm à 7 mm.

10. Dispositif selon l'une des revendications 1 à 9,
**caractérisé en ce que**
le tuyau de cathéter (3) est réalisé, au niveau de la zone terminale distale dans laquelle le filtre (2) peut être reçu, avec une circonférence plus grande que dans sa zone restante, de sorte que le diamètre du lumen de cathéter est essentiellement constant sur la totalité de la longueur du tuyau de cathéter (3).

11. Dispositif selon l'une des revendications 1 à 10,
**caractérisé en ce que** l'extrémité proximale du tuyau de cathéter (3) est réalisée en vue d'être connectée à un appareil coeur-poumon et/ou à un filtre extérieur.

12. Dispositif selon l'une des revendications 1 à 11,
**caractérisé en ce qu'**une vanne à usage unique est agencée dans le lumen de cathéter (5) du tuyau de cathéter (3), de préférence à l'extrémité proximale du cathéter-filtre, à travers laquelle il est possible de faire passer un autre cathéter.

13. Dispositif selon l'une des revendications 9 à 12,
**caractérisé en ce que**
l'autre cathéter est un cathéter à ballon (16), que l'on peut faire passer à travers le lumen de cathéter (5) du tuyau de cathéter (3) de l'extrémité proximale vers l'extrémité distale, ledit cathéter à ballon (16) étant pourvu à son extrémité distale d'un ballon destiné à l'élimination d'une sténose des valvules cardiaques.

14. Dispositif selon l'une des revendications 1 à 13, **caractérisé en ce que** le filtre (2) est réalisé sous forme allongée et se transforme à son extrémité proximale en un tuyau de cathéter (3), dans lequel le filtre (2) est susceptible d'être déployé hors du cathéter en direction distale, et le filtre (2) est plus long qu'un tronçon de la crosse de l'aorte dans lequel les artères destinées à l'alimentation du cerveau et des bras sont ramifiées, désigné dans ce qui suit comme "tronçon de crosse d'aorte", et le filtre (2) est réalisé de telle façon que, dans la zone en amont de ces aortes, il s'applique de façon étanche contre l'aorte montante au moins par sa zone terminale distale, et soit le filtre est réalisé en se rétrécissant depuis la position distale vers la position proximale, soit le filtre comprend un tronçon de filtre distal (6) de forme tubulaire et un tronçon proximal (7) qui va en se rétrécissant depuis le tronçon de filtre distal (6) vers le tuyau de cathéter (3).

15. Dispositif selon la revendication 14,
**caractérisé en ce que**
le filtre (2) comporte des ouvertures de filtre avec un diamètre d'environ 150 à 250 µm.
